# EUROPEAN PATENT SPECIFICATION

(11) **EP 1 272 200 B1**
(45) Date of publication and mention of the grant of the patent: **22.06.2005**
(21) Application number: 01915495.4
(22) Date of filing: 27.03.2001
(51) Int. Cl.: A61K 31/661, A61P 35/00

(54) **DIVIDED DOSE THERAPIES WITH VASCULAR DAMAGING ACTIVITY**
GETRENNTE DOSIS THERAPIEN MIT GEFÄSSSCHÄDIGENDER AKTIVITÄT
THERAPIES EN DOSES FRACTIONNEES AYANT UN EFFET DE DEGRADATION VASCULAIRE

(30) Priority: 31.03.2000 GB 0007740; 08.06.2000 GB 0013928; 20.06.2000 GB 0014904
(43) Date of publication of application: 08.01.2003
(73) Proprietor: Angiogene Pharmaceuticals Ltd, Oxfordshire OX4 4GA (GB)
(72) Inventor: DAVIS, Peter David, Watlington, Oxfordshire OX9 5SX (GB)
(74) Representative: Burns, Tracy Anne
(86) International application number: PCT/GB2001/001329
(87) International publication number: WO 2001/074369

(56) References cited:
- EP-A- 0 086 554
- WO-A-91/06668
- WO-A-91/15495
- WO-A-92/21660
- WO-A-99/02166
- BREWER G J ET AL: "Treatment of metastatic cancer with tetrathiomolybdate, an anticopper, antiangiogenic agent: Phase I study." CLINICAL CANCER RESEARCH, (2000 JAN) 6 (1) 1-10. , XP000906939
- DATABASE WPI Section Ch, Week 199944 Derwent Publications Ltd., London, GB; Class B01, AN 1999-522702 XP002174902 & JP 11 228594 A (MEIJI MILK PROD CO LTD), 24 August 1999 (1999-08-24)
- DAVIS P D ET AL: "Anti-tumour activity of vascular targeting agent ZD6126 is enhanced by split dosing." CLINICAL CANCER RES., CODEN CCREF ISSN: 1078-0432, vol. 6 (SUPPL., 4522s, Abs. No. 282), November 2000 (2000-11), XP000999405 Angiogene Pharmaceuticals

## Description

The present invention relates to the use of a vascular damaging agent or a pharmaceutically acceptable salt thereof in the manufacture of a medicament for administration in divided doses for use in the production of a vascular damaging effect in a warm-blooded animal such as a human, particularly a use for the treatment of a cancer involving a solid tumour. The present invention particularly relates to such a use wherein the vascular damaging agent is ZD6126.

Normal angiogenesis plays an important role in a variety of processes including embryonic development, wound healing and several components of female reproductive function. Undesirable or pathological angiogenesis has been associated with disease states including diabetic retinopathy, psoriasis, cancer, rheumatoid arthritis, atheroma, Kaposi's sarcoma and haemangioma (Fan et al, 1995, Trends Pharmacol. Sci. 16: 57-66; Folkman, 1995, Nature Medicine 1: 27-31). Formation of new vasculature by angiogenesis is a key pathological feature of several diseases (J. Folkman, New England Journal of Medicine 333, 1757-1763 (1995)). For example, for a solid tumour to grow it must develop its own blood supply upon which it depends critically for the provision of oxygen and nutrients; if this blood supply is mechanically shut off the tumour undergoes necrotic death. Neovascularisation is also a clinical feature of skin lesions in psoriasis, of the invasive pannus in the joints of rheumatoid arthritis patients and of atherosclerotic plaques. Retinal neovascularisation is pathological in macular degeneration and in diabetic retinopathy.

Reversal of neovascularisation by damaging the newly-formed vascular endothelium is expected to have a beneficial therapeutic effect. A number of vascular damaging agents (also known as vascular targeting agents) have been identified, for example combretastatin A4 phosphate and the Ajinomoto compound AC-7700 (Nihei Y. *et al*. Japanese Journal of Cancer Research, 1999, 90, 1016-1025).

It has been found that the compounds of:
International Patent Application No. PCT/GB98/01977 (Publication No. WO 99/02166) and
International Patent Application No. PCT/GB99/04436 (Publication No. WO 00/40529), both of which describe tricyclic compounds; and
International Patent Application No. PCT/GB/00099 (Publication No. WO 00/41669), which describes heteroaromatic compounds;
have a selective damaging effect on newly formed vasculature as compared to the normal, established vascular endothelium of the host species. This is a property of value in the treatment of disease states associated with angiogenesis such as cancer, diabetes, psoriasis, rheumatoid arthritis, Kaposi's sarcoma, haemangioma, acute and chronic nephropathies, atheroma, arterial restenosis, autoimmune diseases, acute inflammation, excessive scar formation and adhesions, endometriosis, dysfunctional uterine bleeding and ocular diseases with retinal vessel proliferation.

One compound described in PCT/GB98/01977 (Publication No. WO 99/02166) is N-acetylcolchinol-O-phosphate, (also know as (5S)-5-(acetylamino)-9,10,11-trimethoxy-6,7-dihydro-5H-dibenzo[a,c]cyclohepten-3-yl dihydrogen phosphate; Example 1 of WO 99/02166), which. is referred to herein as ZD6126.

It is believed, though this is not limiting on the invention, that ZD6126 damages newly-formed vasculature, for example the vasculature of tumours, thus effectively reversing the process of angiogenesis. This may be compared with other known anti-angiogenic agents which tend to be less effective once the vasculature has formed.

Unexpectedly and surprisingly we have now found that vascular damaging agents, such as ZD6126, when dosed in divided doses (also known as split doses) produce a greater anti-tumour effect than when a single dose of the agent is given.

Anti-tumour effects of the use according to the present invention include but are not limited to, inhibition of tumour growth, tumour growth delay, regression of tumour, shrinkage of tumour, increased time to regrowth of tumour on cessation of treatment, slowing of disease progression. It is expected that when the present invention is used in a method of treatment and is administered to a warm-blooded animal such as a human, in need of treatment for cancer involving a solid tumour, said method of treatment will produce an effect, as measured by, for example, one or more of: the extent of the anti-tumour effect, the response rate, the time to disease progression and the survival rate.

According to the present invention there is provided the use of a vascular damaging agent or a pharmaceutically acceptable salt thereof in the manufacture of a medicament for administration in divided doses for use in the production of a vascular damaging effect in a warm-blooded animal such as a human wherein the total dose of the vascular damaging agent, to be administered in any one day period, is divided into two or more fractions which are about equal or unequal, with a time period between each fraction of greater than 0 hours to about 6 hours; and
wherein the vascular damaging effect produced using divided doses of the vascular damaging agent is greater than the effect produced by administering the same total dose of the vascular damaging agent as a single dose.

According to a further aspect of the present invention there is provided the use of a vascular damaging agent or a pharmaceutically acceptable salt thereof in the manufacture of a medicament for administration in divided doses for use in the production of an anti-cancer effect in a warm-blooded animal such as a human.

According to a further aspect of the present invention there is provided the use of a vascular damaging agent or a pharmaceutically acceptable salt thereof in the manufacture of a medicament for administration in divided doses for use in the production of an anti-tumour effect in a warm-blooded animal such as a human.

According to a further aspect of the present invention there is provided the use of ZD6126 or a pharmaceutically acceptable salt thereof in the manufacture of a medicament for administration in divided doses for use in the production of a vascular damaging effect in a warm-blooded animal such as a human.

According to a further aspect of the present invention there is provided the use of ZD6126 or a pharmaceutically acceptable salt thereof in the manufacture of a medicament for administraton in divided doses for use in the production of an anti-cancer effect in a warm-blooded animal such as a human.

According to a further aspect of the present invention there is provided the use of ZD6126 or a pharmaceutically acceptable salt thereof in the manufacture of a medicament for administration in divided doses for use in the production of an anti-tumour effect in a warm-blooded animal such as a human.

The present invention may be used in a method for treatment of a cancer involving a solid tumour in a warm-blooded animal such as a human which comprises administering to said animal in divided doses an effective amount of a vascular damaging agent or a pharmaceutically acceptable salt thereof.

The invention may be used in a method for the production of a vascular damaging effect in a warm-blooded animal such as a human, which comprises administering to said animal in divided doses an effective amount of ZD6126: 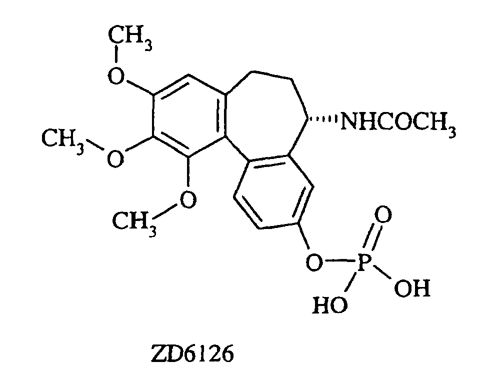 or a pharmaceutically acceptable salt thereof.

The invention may be used in a method for the treatment of a cancer involving a solid tumour in a warm-blooded animal such as a human, which comprises administering to said animal in divided doses an effective amount of ZD6126 or a pharmaceutically acceptable salt thereof.

The invention may be used in a method for the production of a vascular damaging effect in a warm-blooded animal such as a human, which comprises administering to said animal in divided doses an effective amount of a vascular damaging agent or a pharmaceutically acceptable salt thereof wherein the vascular damaging agent or a pharmaceutically acceptable salt thereof may optionally be administered together with a pharmaceutically acceptable excipient or carrier.

The invention may be used in a method for the treatment of a cancer involving a solid tumour in a warm-blooded animal such as a human, which comprises administering to said animal in divided doses an effective amount of a vascular damaging agent or a pharmaceutically acceptable salt thereof wherein the vascular damaging agent or a pharmaceutically acceptable salt thereof may optionally be administered together with a pharmaceutically acceptable excipient or carrier.

The invention may be used in a method for the production of a vascular damaging effect in a warm-blooded animal such as a human, which comprises administering to said animal in divided doses an effective amount of ZD6126 or a pharmaceutically acceptable salt thereof wherein ZD6126 or a pharmaceutically acceptable salt thereof may optionally be administered together with a pharmaceutically acceptable excipient or carrier.

The invention may be used in a method for the treatment of a cancer involving a solid tumour in a warm-blooded animal such as a human, which comprises administering to said animal in divided doses an effective amount of ZD6126 or a pharmaceutically acceptable salt thereof wherein ZD6126 or a pharmaceutically acceptable salt thereof may optionally be administered together with a pharmaceutically acceptable excipient or carrier.

The use of invention in a method of treatment using divided doses of a vascular damaging agent, such as ZD6126, is expected to be significantly greater than the effect of a method of treatment using a single dose of vascular damaging agent such as ZD6126.

Vascular damaging agents (VDAs) are agents which damage vasculature especially newly formed vasculature such as tumour vasculature.
Preferred VDAs are those described in International Patent Application No. PCT/GB98/01977 (Publication No. WO 99/02166).
Other preferred VDAs are those described in International Patent Application No. PCT/GB99/04436 (Publication No. WO 00/40529).
Other preferred VDAs are those described in International Patent Application No. PCT/GB/00099 (Publication No. WO 00/41669).
Another VDA is combretastatin A4 phosphate.
Another VDA is the Ajinomoto compound AC-7700 (Nihei Y. *et al*. Japanese Journal of Cancer Research, 1999, 90, 1016-1025).
An especially preferred VDA is ZD6126.

A vascular damaging agent may be in a form suitable for oral administration, for example as a tablet or capsule, for nasal administration or administration by inhalation, for example as a powder or solution, for parenteral injection (including intravenous, subcutaneous, intramuscular, intravascular or infusion) for example as a sterile solution, suspension or emulsion, for topical administration for example as an ointment or cream, for rectal administration for example as a suppository or the route of administration may be by direct injection into the tumour or by regional delivery or by local delivery. In other embodiments of the present invention the VDA of the method of treatment may be delivered endoscopically, intratracheally, intralesionally, percutaneously, intravenously, subcutaneously, intraperitoneally or intratumourally. The VDA may be in the form of a pharmaceutical composition wherein the VDA or a pharmaceutically acceptable salt thereof is in association with a pharmaceutically acceptable excipient or carrier. In general the compositions described herein may be prepared in a conventional manner using conventional excipients. The compositions of the present invention are advantageously presented in unit dosage form.

Divided doses, also called split doses, means that the total dose to be administered to a warm-blooded animal, such as a human, in any one day period (for example one 24 hour period from midnight to midnight) is divided up into two or more fractions of the total dose and these fractions are administered with a time period between each fraction of about greater than 0 hours to about 6 hours, preferably about 1 hour to about 6 hours, more preferably about 2 hours to about 4 hours. The fractions of total dose may be about equal or unequal. Preferably the total dose is divided into two parts which may be about equal or unequal. The time intervals between doses may be for example selected from:
about 1 hour, about 1.5 hours, about 2 hours, about 2.5 hours, about 3 hours, about 3.5 hours, about 4 hours, about 4.5 hours, about 5 hours, about 5.5 hours and about 6 hours. If more than two doses are administered the time intervals between each dose may be about equal or unequal.
Preferably two doses are given with a time interval in between them of greater than or equal to 1 hour and less than 6 hours. More preferably two doses are given with a time interval in between them of greater than or equal to two hours and less than 5 hours.
Yet more preferably two doses are given with a time interval in between them of greater than or equal to two hours and less than or equal to 4 hours.
Particularly the total dose is divided into two parts which may be about equal or unequal with a time interval between doses of greater than or equal to about two hours and less than or equal to about 4 hours.
More particularly the total dose is divided into two parts which may be about equal with a time interval between doses of greater than or equal to about two hours and less than or equal to about 4 hours.
For the avoidance of doubt the term 'about' in the description of time periods means the time given plus or minus 15 minutes, thus for example about 1 hour means 45 to 75 minutes, about 1.5 hours means 75 to 105 minutes. Elsewhere the term 'about' has its usual dictionary meaning.

ZD6126 will normally be administered to a warm-blooded animal at a unit dose within the range 10-500mg per square metre body area of the animal, for example approximately 0.3-15mg/kg in a human. A unit dose in the range, for example, 0.3-15mg/kg, preferably 0.5-5mg/kg is envisaged and this is normally a therapeutically-effective dose. A unit dosage form such as a tablet or capsule will usually contain, for example 25-250mg of active ingredient. Preferably a daily dose in the range of 0.5-5mg/kg is employed.

As stated above the size of the total daily dose which is required for the therapeutic or prophylactic treatment of a particular disease state will necessarily be varied depending on the host treated, the route of administration and the severity of the illness being treated. Accordingly the optimum dosage may be determined by the practitioner who is treating any particular patient. For example, it may be necessary or desirable to reduce the above-mentioned doses of the treatment in order to reduce toxicity.

The methods of treatment of the present invention as defined herein may be applied as a sole therapy or may involve, in addition to a vascular damaging agent administered in divided doses, one or more other substances and/or treatments. Such conjoint treatment may be achieved by way of the simultaneous, sequential or separate administration of the individual components of the treatment. In the field of medical oncology it is normal practice to use a combination of different forms of treatment to treat each patient with cancer. In medical oncology the other component(s) of such conjoint treatment in addition to the VDA administered in divided doses, may be: surgery, radiotherapy or chemotherapy. Such chemotherapy may include the following categories of therapeutic agent:
(i) antiangiogenic agents (for example linomide, inhibitors of integrin αvβ3 function, angiostatin, endostatin, razoxin, thalidomide) and including vascular endothelial growth factor (VEGF) receptor tyrosine kinase inhibitors (RTKIs) (for example those described in International Patent Applications Publication Nos. WO 97/22596, WO 97/30035, WO 97/32856 and WO 98/13354, also for example those described in International Patent Application Publication No. WO 00/47212);
(ii) cytostatic agents such as antioestrogens (for example tamoxifen, toremifene, raloxifene, droloxifene, iodoxyfene), progestogens (for example megestrol acetate), aromatase inhibitors (for example anastrozole, letrazole, vorazole, exemestane), antiprogestogens, antiandrogens (for example flutamide, nilutamide, bicalutamide, cyproterone acetate), LHRH agonists and antagonists (for example goserelin acetate, luprolide), inhibitors of testosterone 5α-dihydroreductase (for example finasteride), anti-invasion agents (for example metalloproteinase inhibitors like marimastat and inhibitors of urokinase plasminogen activator receptor function) and inhibitors of growth factor function, (such growth factors include for example epidermal growth factor (EGF), platelet derived growth factor and hepatocyte growth factor such inhibitors include growth factor antibodies, growth factor receptor antibodies, tyrosine kinase inhibitors and serine/threonine kinase inhibitors);
(iii) biological response modifiers (for example interferon);
(iv) antibodies (for example edrecolomab); and
(v) antiproliferative/antineoplastic drugs and combinations thereof, as used in medical oncology, such as antimetabolites (for example antifolates like methotrexate, fluoropyrimidines like 5-fluorouracil, purine and adenosine analogues, cytosine arabinoside); antitumour antibiotics (for example anthracyclines like doxorubicin, daunomycin, epirubicin and idarubicin, mitomycin-C, dactinomycin, mithramycin); platinum derivatives (for example cisplatin, carboplatin); alkylating agents (for example nitrogen mustard, melphalan, chlorambucil, busulphan, cyclophosphamide, ifosfamide, nitrosoureas, thiotepa); antimitotic agents (for example vinca alkaloids like vincristine and taxoids like taxol, taxotere); enzymes (for example asparaginase); thymidylate synthase inhibitors (for example raltitrexed); topoisomerase inhibitors (for example epipodophyllotoxins like etoposide and teniposide, amsacrine, topotecan, irinotecan).

Where the VDA is ZD6126, salts for use in pharmaceutical compositions will be pharmaceutically acceptable salts, but other salts may be useful in the production of ZD6126 and its pharmaceutically acceptable salts. Such salts may be formed with an inorganic or organic base which affords a pharmaceutically acceptable cation. Such salts with inorganic or organic bases include for example an alkali metal salt, such as a sodium or potassium salt, an alkaline earth metal salt such as a calcium or magnesium salt, an ammonium salt or for example a salt with methylamine, dimethylamine, trimethylamine, piperidine, morpholine or tris-(2-hydroxyethyl)amine.

ZD6126 may be made according to the following process.

N-Acetylcolchinol (30.0g, 83.9mmol) is dissolved in acetonitrile under an inert atmosphere and 1,2,3-triazole (14.67g, 212.4mmol) added via a syringe. Di-tert-butyl-diethylphosphoramidite (37.7g, 151.4mmol) is added and the reaction mixture stirred at about 20°C to complete the formation of the intermediate phosphite ester. Cumene hydroperoxide (24.4g, 159.2mmol) is added at about 10°C and the reaction mixture stirred until the oxidation is complete. Butyl acetate (50ml) and sodium hydroxide solution (250ml of 1M) are added, the reaction mixture stirred and the aqueous phase discarded. The organic solution is washed with sodium hydroxide solution (2 x 250ml of 1M) and a saturated solution of sodium chloride. Trifluoroacetic acid (95.3g, 836mmol) is added at about 15°C. The reaction mixture is distilled at atmospheric pressure, ZD6126 crystallises and is isolated at ambient temperature.

### Cell Survival Assay

The activity of ZD6126 administered in split doses may be demonstrated by the following cell survival assay.
In vivo cell survival was measured using an excision assay (D J Chaplin et al., Anticancer Research 19: 189-196 (1999)).
For each of the assays a) and b) below, the surviving fraction of tumour cells was determined as follows:
Tumours were excised at about 18 hours after treatment, weighed and disaggregated for 1 hour at 37 degrees Celsius in an enzyme cocktail containing 1mg/ml pronase, 0.5 mg/ml DNAase and 0.5 mg/ml collagenase. Haemocytometer counts of trypan blue-excluding cells were made and viable cells seeded in appropriate concentrations to yield about 50 colonies/dish after in vitro incubation. Heavily irradiated feeder cells (V79 cells) were used at a concentration of 25,000/ml to support the growth of the surviving CaNT cells. The data were calculated as surviving fraction per gram of tumour.

### a) CaNT Tumour Model: Effect of Dosage Interval

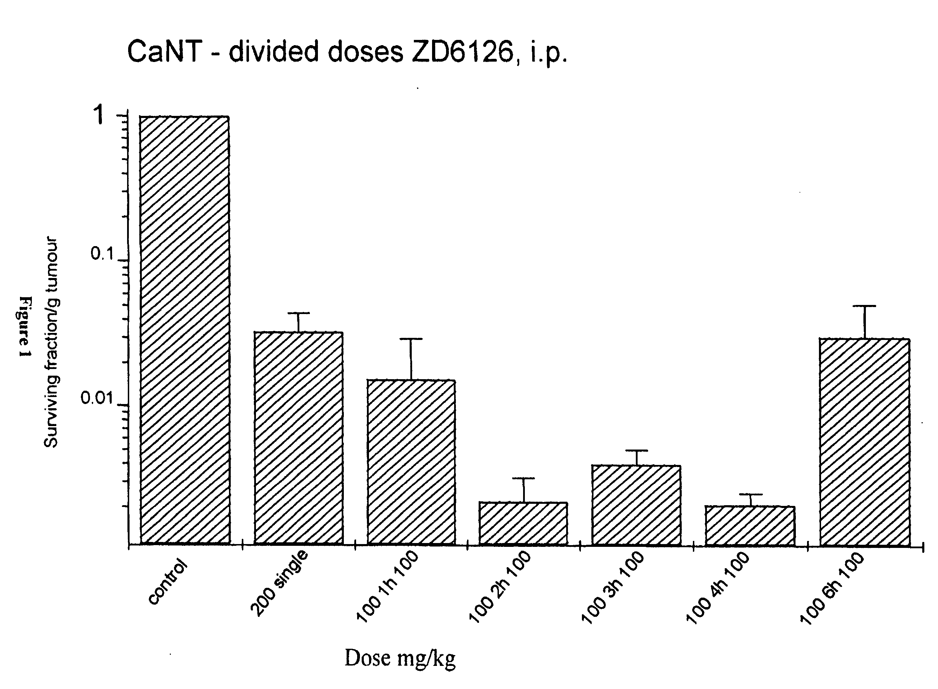

In the murine adenocarcinoma CaNT tumour model grown in female CBA mice (Hill, S.A et al, Int. J. Cancer 63, 119-123, 1995) administering ZD6126 in divided doses resulted in an improved anti-tumour effect compared to ZD6126 administered as a single dose as measured by surviving fraction of tumour cells. See Figure 1.

### Methodology

### Single Dose

ZD6126 was administered as a single dose of 200mg intra-peritoneally (i.p.) in saline with a small amount of 1% sodium carbonate added to aid the dissolution of ZD6126.

### Divided Doses

ZD6126 was dosed using a split dose regimen of 100mg/kg ZD6126, followed by a time interval, followed by a further 100mg/kg ZD6126; doses were given intraperitoneally (i.p.) in saline with a small amount of 1% sodium carbonate added to aid the dissolution of ZD6126.
The time intervals used were 1, 2, 3, 4 and 6 hours.
Surviving fraction per gram of tumour was determined as described above and plotted as shown in Figure 1.
Two doses of 100mg/kg separated by 2, 3 or 4 hours were significantly more effective in this model than a single 200mg/kg dose.

### b) CaNT Tumour Model: Effect of Dosage Interval and Split Dose Proportions

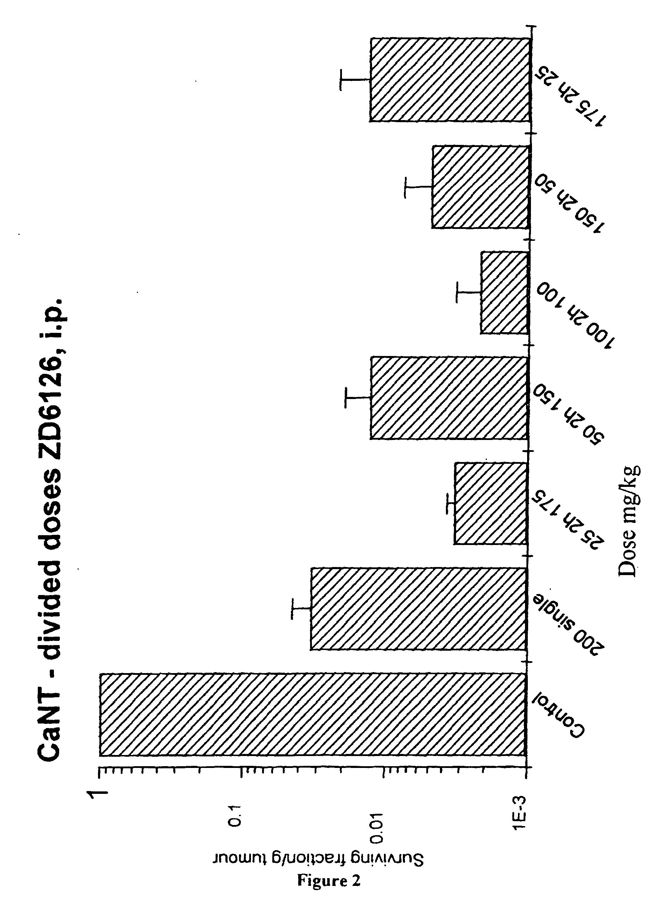

In the murine adenocarcinoma CaNT tumour model grown in female CBA mice (Hill, S.A et al, Int. J. Cancer 63, 119-123, 1995) administering ZD6126 in divided doses 2 hours apart resulted in an improved anti-tumour effect, as measured by surviving tumour cell fraction, compared to ZD6126 administered as a single dose. This improved effect varied with the proportion of total dose given in the first and second doses. See Figure 2.

### Methodology

### Single Dose

ZD6126 was administered as a single dose of 200mg intra-peritoneally (i.p.) in saline with a small amount of 1% sodium carbonate added to aid the dissolution of ZD6126.

### Divided Doses

ZD6126 was dosed using split dose regimens of:
i) 25mg/kg ZD6126, followed by a 2 hour interval, followed by a further 175mg/kg ZD6126;
ii) 50mg/kg ZD6126, followed by a 2 hour interval, followed by a further 150mg/kg ZD6126;
iii) 100mg/kg ZD6126, followed by a 2 hour interval, followed by a further 100mg/kg ZD6126;
iv) 150mg/kg ZD6126, followed by a 2 hour interval, followed by a further 50mg/kg ZD6126;
v) 175mg/kg ZD6126, followed by a 2 hour interval, followed by a further 25mg/kg ZD6126;
All doses were given intraperitoneally (i.p.) in saline with a small amount of 1% sodium carbonate added to aid the dissolution of ZD6126.

The anti-tumour effect, as measured by surviving fraction of tumour cells, was greater with divided doses of ZD6126 than with a single dose of 200mg/kg ZD6126. This greater effect was significant when divided doses of ZD6126 were administered according to i), iii) or iv) above. The best effect was seen with equal split doses, ie according to iii) above.

## Claims

1. Use of a vascular damaging agent or a pharmaceutically acceptable salt thereof in the manufacture of a medicament for administration in divided doses for use in the production of a vascular damaging effect in a warm-blooded animal such as a human wherein the total dose of the vascular damaging agent, to be administered in any one day period, is divided into two or more fractions which are about equal or unequal, with a time period between each fraction of greater than 0 hours to about 6 hours; and
wherein the vascular damaging effect produced using divided doses of the vascular damaging agent is greater than the effect produced by administering the same total dose of the vascular damaging agent as a single dose.

2. Use according to claim 1 wherein the interval between fractions is greater than or equal to about 1 hour and less than about 6 hours.

3. Use according to claim 1 wherein the interval between fractions is greater than or equal to 2 hours and less than 5 hours.

4. Use according to claim 1 wherein the interval between fractions is greater than or equal to about 2 hours and less than or equal to about 4 hours.

5. Use according to any one of the preceding claims wherein the total dose of the vascular damaging agent, to be administered in any one day period, is divided into two parts which are about equal or unequal.

6. Use according to claim 4 wherein the total dose of the vascular damaging agent, to be administered in any one day period, is divided into two parts which are about equal.

7. Use according to any one of claims 1-6 wherein the medicament is for use in the production of an anti-cancer effect in a warm-blooded animal such as a human.

8. Use according to any one of claims 1-6 wherein the medicament is for use in the production of an anti-tumour effect in a warm-blooded animal such as a human.

9. Use according to any one of claims 1-6 wherein the medicament is for use in the treatment of a cancer involving a solid tumour in a warm-blooded animal such as a human.

10. Use according to any one of claims 1-9 wherein the vascular damaging agent is ZD6126: 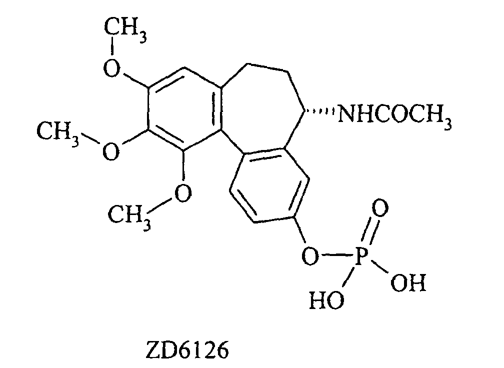 or a pharmaceutically acceptable salt thereof.

11. Use according to any one of claims 1-9 wherein the vascular damaging agent is combretastatin A4 phosphate.

12. Use according to any one of claims 1-9 wherein the vascular damaging agent is AC-7700.

## Patentansprüche

1. Verwendung eines gefäßschädigenden Mittels oder eines pharmazeutisch annehmbaren Salzes davon zur Herstellung eines Medikaments zur Verabreichung in Teildosen zur Verwendung beim Hervorrufen einer gefäßschädigenden Wirkung in einem Warmblüter wie dem Menschen, wobei die Gesamtdosis an im Verlauf eines Tages zu verabreichendem gefäßschädigenden Mittel in zwei oder mehr Teile geteilt wird, die etwa gleich oder nicht gleich sind, wobei die Zeitspanne zwischen den Dosen mehr als 0 Stunden bis etwa 6 Stunden beträgt, und
wobei die durch die Verwendung der Teildosen des gefäßschädigenden Mittels hervorgerufene gefäßschädigende Wirkung größer ist als die durch Verabreichung der gleichen Gesamtdosis des gefäßschädigenden Mittels als Einzeldosis hervorgerufene Wirkung.

2. Verwendung nach Anspruch 1, wobei die Zeitspanne zwischen den Teildosen mehr als oder gleich etwa 1 Stunde und weniger als etwa 6 Stunden beträgt.

3. Verwendung nach Anspruch 1, wobei die Zeitspanne zwischen den Teildosen mehr als oder gleich 2 Stunden und weniger als 5 Stunden beträgt.

4. Verwendung nach Anspruch 1, wobei die Zeitspanne zwischen den Teildosen mehr als oder gleich etwa 2 Stunden und weniger als oder gleich etwa 4 Stunden beträgt.

5. Verwendung nach einem der vorhergehenden Ansprüche, wobei die Gesamtdosis an im Verlauf eines Tages zu verabreichendem gefäßschädigenden Mittel in zwei Teile geteilt wird, die etwa gleich oder ungleich sind.

6. Verwendung nach Anspruch 4, wobei die Gesamtdosis an im Verlauf eines Tages zu verabreichendem gefäßschädigenden Mittel in zwei Teile geteilt wird, die etwa gleich sind.

7. Verwendung nach einem der Ansprüche 1-6, wobei das Medikament zum Hervorrufen einer Antikrebswirkung in einem Warmblüter wie dem Menschen verwendet wird.

8. Verwendung nach einem der Ansprüche 1-6, wobei das Medikament zum Hervorrufen einer Antitumorwirkung in einem Warmblüter wie dem Menschen verwendet wird.

9. Verwendung nach einem der Ansprüche 1-6, wobei das Medikament zur Behandlung einer Krebserkrankung, bei der ein fester Tumor vorliegt, in einem Warmblüter wie dem Menschen verwendet wird.

10. Verwendung nach einem der Ansprüche 1-9, wobei es sich bei dem gefäßschädigenden Mittel um ZD6126: 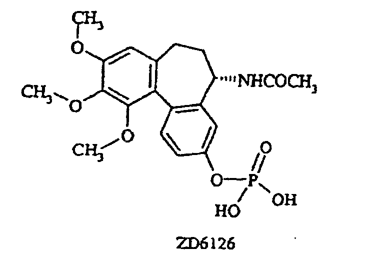 oder ein pharmazeutisch annehmbares Salz davon handelt.

11. Verwendung nach einem der Ansprüche 1-9, wobei es sich bei dem gefäßschädigenden Mittel um Combretastatin-A4-phosphat handelt.

12. Verwendung nach einem der Ansprüche 1-9, wobei es sich bei dem gefäßschädigenden Mittel um AC-7700 handelt.

## Revendications

1. Utilisation d'un agent de dégradation vasculaire ou d'un sel pharmaceutiquement acceptable de celui-ci pour la fabrication d'un médicament destiné à une administration en doses fractionnées, pour une utilisation dans la production d'un effet de dégradation vasculaire chez un animal à sang chaud tel qu'un être humain, dans laquelle la dose totale de l'agent de dégradation vasculaire destinée à être administrée sur une période quelconque d'une journée, est fractionnée en deux fractions ou plus qui sont approximativement égales ou inégales avec une période de temps entre chaque fraction de plus de 0 à environ 6 heures ; et
dans laquelle l'effet de dégradation vasculaire produit en utilisant des doses fractionnées de l'agent de dégradation vasculaire est supérieur à l'effet produit par l'administration de la même dose totale de l'agent de dégradation vasculaire en tant que dose unique.

2. Utilisation selon la revendication 1, dans laquelle l'intervalle entre les fractions est supérieur ou égal à environ 1 heure et inférieur à environ 6 heures.

3. Utilisation selon la revendication 1, dans laquelle l'intervalle entre les fractions est supérieur ou égal à 2 heures et inférieur à 5 heures.

4. Utilisation selon la revendication 1, dans laquelle l'intervalle entre les fractions est supérieur ou égal à environ 2 heures et inférieur ou égal à environ 4 heures.

5. Utilisation selon l'une quelconque des revendications précédentes, dans laquelle la dose totale de l'agent de dégradation vasculaire destinée à être administrée sur une période quelconque d'une journée, est divisée en deux parties qui sont approximativement égales ou inégales.

6. Utilisation selon la revendication 4, dans laquelle la dose totale de l'agent de dégradation vasculaire destinée à être administrée sur une période quelconque d'une journée, est divisée en deux parties qui sont approximativement égales.

7. Utilisation selon l'une quelconque des revendications 1 à 6, dans laquelle le médicament est destiné à être utilisé pour la production d'un effet anticancéreux chez un animal à sang chaud tel qu'un être humain.

8. Utilisation selon l'une quelconque des revendications 1 à 6, dans laquelle le médicament est destiné à être utilisé pour la production d'un effet antitumoral chez un animal à sang chaud tel qu'un être humain.

9. Utilisation selon l'une quelconque des revendications 1 à 6, dans laquelle le médicament est destiné à être utilisé pour le traitement d'un cancer impliquant une tumeur solide chez un animal à sang chaud tel qu'un être humain.

10. Utilisation selon l'une quelconque des revendications 1 à 9, dans laquelle l'agent de dégradation vasculaire est ZD6126 : 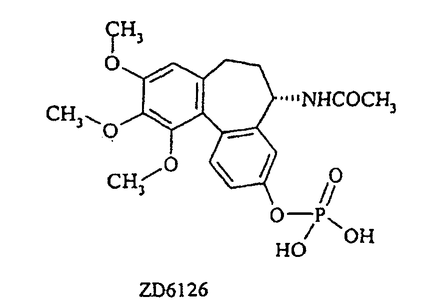 ou un sel pharmaceutiquement acceptable de celui-ci.

11. Utilisation selon l'une quelconque des revendications 1 à 9, dans laquelle l'agent de dégradation vasculaire est le phosphate de combretastatine A4.

12. Utilisation selon l'une quelconque des revendications 1 à 9, dans laquelle l'agent de dégradation vasculaire est AC-7700.
